# EUROPEAN PATENT SPECIFICATION

(11) **EP 2 971 069 B1**
(45) Date of publication and mention of the grant of the patent: **17.10.2018**
(21) Application number: 13712642.1
(22) Date of filing: 13.03.2013
(51) Int. Cl.: C12Q 1/6869, G06F 19/22

(54) **METHODS AND SYSTEMS FOR ALIGNING REPETITIVE DNA ELEMENTS**
VERFAHREN UND SYSTEME ZUM AUSRICHTEN WIEDERHOLTER DNA ELEMENTE
PROCÉDÉS ET SYSTÈMES POUR ALIGNER DES ÉLÉMENTS D'ADN RÉPÉTITIFS

(43) Date of publication of application: 20.01.2016
(73) Proprietor: Illumina, Inc., San Diego, CA 92122 (US)
(72) Inventor: BRUAND, Jocelyne, San Diego, California 92122 (US); RICHARDSON, Tom, San Diego, California 92122 (US); MANN, Tobias, MI 48103 (US)
(74) Representative: Murgitroyd & Company
(86) International application number: PCT/US2013/030867
(87) International publication number: WO 2014/142831

(56) References cited:
- US-A1- 2003 152 955
- US-A1- 2009 117 542
- GYMREK MELISSA ET AL: "lobSTR: A short tandem repeat profiler for personal genomes.", GENOME RESEARCH JUN 2012, vol. 22, no. 6, June 2012 (2012-06), pages 1154-1162, XP002713504, ISSN: 1549-5469
- SURYA SAHA ET AL: "Computational Approaches and Tools Used in Identification of Dispersed Repetitive DNA Sequences", TROPICAL PLANT BIOLOGY, vol. 1, no. 1, 1 March 2008 (2008-03-01), pages 85-96, XP055080810, ISSN: 1935-9756, DOI: 10.1007/s12042-007-9007-5

## Description

### BACKGROUND

Sets of polymorphic, repetitive DNA elements are useful for many genetic applications including paternity testing, human identification (forensic DNA analysis), chimera monitoring (tissue transplantation monitoring), as well as many other uses in plant and animal genomics. One class of these repetitive elements comprises of the short tandem repeats (STRs). The allele of an STR locus is defined by its length, or number of repeat units, and by its sequence variation. While capillary electrophoresis systems can show the length of the allele, sequencing technologies have the additional differentiation power of discovering sequence variation, such as SNPs.

In order to take advantage of NGS data, it is advantageous to accurately and efficiently assign reads to the correct STR locus and STR allele.

Gymrek M. et al, 2012, Genome Research, 22: 1154-1162, discloses a method for determining the length and/or the sequence of a polymorphic repetitive DNA element having a repeat region situated between a first conserved flanking region and a second conserved flanking region.

Existing methods for alignment of sequencing reads are time consuming and unable to detect all known and undiscovered polymorphic repetitive regions. As such, a great need exists for improved methods and systems for aligning repetitive DNA elements.

### BRIEF SUMMARY

Presented herein are methods and systems for aligning repetitive DNA elements. The methods and systems use the conserved flanks of repetitive polymorphic loci to effectively determine the length and sequence of the repetitive DNA element.

Accordingly, one embodiment presented herein is a method for determining the length and/or sequence of a polymorphic repetitive DNA element having a repeat region situated between a first conserved flanking region and a second conserved flanking region, the method comprising: (a) providing a data set comprising at least one sequence read of the polymorphic repetitive DNA element; (b) providing a reference sequence comprising the first conserved flanking region and the second conserved flanking region; (c) aligning a portion of the first flanking region of the reference sequence to the sequence read; (d) aligning a portion of the second flanking region of the reference sequence to the sequence read; and (e) determining the length and/or sequence of the repeat region; wherein at least steps (c), (d) and (e) are performed using a suitably programmed computer; wherein the aligning a portion of the flanking region in one or both of steps (c) and (d) comprises: (i) determining a location of a conserved flanking region on the read by using exact k-mer matching of a seeding region which overlaps or is adjacent to the repeat region; and (ii) aligning the flanking region to the sequence read; wherein the seeding region comprises a high-complexity region of the conserved flanking region, the high-complexity region comprising sequence that is sufficiently distinct from the repeat region so as to avoid mis-alignment. In some embodiments, the aligning can further comprise aligning both the flanking sequence and a short adjacent region comprising a portion of the repeat region.

Also presented herein is a system for determining the length and/or sequence of a polymorphic repetitive DNA element having a repeat region situated between a first conserved flanking region and a second conserved flanking region, the system comprising: a processor; and a program for determining the length and/or sequence of a polymorphic repetitive DNA element, the program comprising instructions for the processor to perform the following steps: (a) providing a data set comprising at least one sequence read of the polymorphic repetitive DNA element; (b) providing a reference sequence comprising the first conserved flanking region and the second conserved flanking region; (c) aligning a portion of the first flanking region of the reference sequence to the sequence read; (d) aligning a portion of the second flanking region of the reference sequence to the sequence read; and (e) determining the length and/or sequence of the repeat region; wherein the aligning a portion of the flanking region in one or both of steps (c) and (d) comprises: (i) determining a location of a conserved flanking region on the read by using exact k-mer matching of a seeding region which overlaps or is adjacent to the repeat region; and (ii) aligning the flanking region to the sequence read; wherein the seeding region comprises a high-complexity region of the conserved flanking region, the high-complexity region comprising sequence that is sufficiently distinct from the repeat region so as to avoid mis-alignment. In some embodiments, the aligning can further comprise aligning both the flanking sequence and a short adjacent region comprising a portion of the repeat region.

In certain embodiments of the above methods or systems, the seeding region comprises a high-complexity region of the conserved flanking region, for example, the high-complexity region comprising a sequence having a diverse mixture of bases. In some embodiments, the seeding region avoids low-complexity regions of the conserved flanking region, for example sequence that substantially resembles that of the repeat sequence and/or sequence having a mixture of bases with low diversity.

In certain embodiments of the above methods or systems, the seeding region is directly adjacent to the repeat region and/or comprises a portion of the repeat region. In certain embodiments, the seeding region is offset from the repeat region.

In certain embodiments of the above methods or systems, the dataset of sequence reads comprises sequence data from a PCR amplicon having a forward and reverse primer sequence. In certain embodiments, the at least one sequence read in the data set comprises a consensus sequence derived from multiple sequence reads. In certain embodiments, providing a reference sequence comprises identifying a locus of interest based upon the primer sequence of the PCR amplicon.

In certain methods or systems, the repeat region is a short tandem repeat (STR) such as, for example, a STR selected from the CODIS autosomal STR loci, CODIS Y-STR loci, EU autosomal STR loci, EU Y-STR loci and the like.

The details of one or more embodiments are set forth in the accompanying drawings and the description below. Other features, objects, and advantages will be apparent from the description and drawings, and from the claims.

### BRIEF DESCRIPTION OF THE DRAWINGS

Figure 1 is a schematic showing a method of alignment according to one embodiment.
Figure 2 is a schematic showing various mis-alignment errors that can occur if the flanking region immediately adjacent to the STR is used to seed the alignment.
Figure 3 is a set of graphs showing actual STR calling compared to theoretical results based on sample input from a mixture of samples.
Figure 4 is a table showing 100% concordance for allele calls for known loci of five control DNA samples.

### DETAILED DESCRIPTION

Sets of polymorphic, repetitive DNA elements are useful for many genetic applications including paternity testing, human identification (forensic DNA analysis), chimera monitoring (tissue transplantation monitoring), as well as many other uses in plant and animal genomics. In order to take advantage of next generation sequencing (NGS) data, tools are needed for accurate and efficient assignment of sequencing reads to the correct repetitive DNA element locus and allele. One class of these repetitive elements comprises of the short tandem repeats (STRs). The allele of an STR locus is defined by its length, or number of repeat units, and by its sequence variation. While capillary electrophoresis systems can show the length of the allele, sequencing technologies have the additional differentiation power of discovering sequence variation, such as SNPs. It will be appreciated that although the methods and systems described herein are discussed in the context of STRs, they can be applied to any other repetitive DNA element.

Existing alignment methods fail for various reasons. One common approach is alignment to a reference sequence is commonly performed. However, the difference in allele sizes greatly differs, even within a single locus. For example, one core U.S. locus, FGA, has known alleles between 12.2 and 51.2, involving differences of 156 nucleotides (or even greater). Most aligners will not align reads with such a large gap, and any alleles which are too far from a reference sequence will be discarded by the aligner.

Another existing approach with drawbacks is the method of aligning to a reference ladder. Typically, a "reference genome" is created by building a ladder of all known STR alleles and aligning the reads to this reference, as typically done with NGS whole genome sequence data or targeted sequencing of non-repetitive DNA regions. There are shortcomings to this method. For example, known information about the STR sequence, such as primer sequence or conserved flanking regions, is ignored. Existing ladders are incomplete, since the sequences of many polymorphic repetitive regions are currently unknown. Due the highly variable nature of these genomic regions, new alleles may be discovered in the future. Further, changes to the sequence of one allele in the reference may have global effects to the reads alignment due to homology between the sequences.

Another alternative methodology for detecting STRs, known as lobSTR, senses then calls all existing STRs from sequencing data of a single sample *de novo,* with no prior knowledge of the STRs (*see* Gymrek et al. 2012 Genome Research 22:1154-62). However, the lobSTR method ignores prior knowledge (primer sequences, flanking regions) and miscalls some alleles. Further lobSTR misses STR loci with complex repeat patterns, including some from the CODIS such as D21S11, allele 24 ([TCTA]₄[TCTG]₆[TCTA]₃TA[TCTA]₃TCA[TCTA]₂TCCA TA[TCTA]₆) or vWA, allele 16 (TCTA[TCTG]₃[TCTA]₁₂TCCA TCTA). Further, lobSTR assumes homozygous or heterozygous alleles, and is therefore not useful for handling samples having mixtures.

Thus, there exists a great need for a targeted approach utilizing prior knowledge greatly increases sensitivity and specificity.

Presented herein are methods and systems which use the conserved flanks of repetitive polymorphic loci to effectively determine the sequence of the repetitive DNA element. The methods advantageously align the beginning of the read sequence to the possible primer sequences to establish the locus and strand to which the read corresponds. Then, sections of the appropriate flanking sequences on each side of the repetitive locus are aligned to the read in order to pull the exact length and sequence from the read. These alignments are seeded using a k-mer strategy. The seed regions can be, for example, in a pre-chosen high-complexity region of the flanking sequence, close to the repeat region, but avoiding low-complexity sequence with homology to the target locus. This approach advantageously avoids misalignment of low-complexity flanking sequences close to the repeat region of interest.

The approach described herein is novel, and is surprisingly effective in properly determining the allele size and sequence. The methods make use of known sequences in the flanks of the STR themselves, which have been previously defined based on the known existing variations among the human population. Advantageously, performing alignment of a short span of flanking regions is computationally quick when compared to other methods. For example, a dynamic programming alignment (Smith-Waterman type) of the entire read is CPU intensive, time consuming, especially where multiple sequence reads are to be aligned. Furthermore, time spent aligning an entire sequence (for which a reference may not even exist) takes up valuable computational resources.

Using flanking regions to properly determine the allele provides several other unexpected advantages over existing methods. For example, BWA, a typical aligner, performs poorly when it is used to align to a reference, primarily due to the repetitive nature of an STR sequence and the incomplete state of the reference. Further, the inventors have observed that changing the reference for one STR locus often affected calls for another locus, which should be independent. However, because forensics applications require high confidence calls, there is very little room for error.

Additional embodiments of the methods provided herein identify unique seeds within a flanking sequence. This approach allows for a reduction in alignment time and plays a role in avoiding misalignments in the case of low-complexity flanks.

The methods presented herein make use of prior knowledge of flanking sequence to ensure the proper call of the STR allele. In contrast, existing methods, which rely on a full reference sequence for each allele, face significant failure rates in situations where there is an incomplete reference. There are many alleles for which the sequence is not known, and possibly some yet unknown alleles. By way of illustration, assume a locus with a simple repeat pattern [TCTA] and a 3' flank starting with the sequence TCAGCTA. Thus, the reference may include such sequences as [flank1][TCTA]ₙTCAGCTA[rest_of_flank2], where n is the number of repeats in the allele. The 9.3 allele would differ from the 10 allele by having a deletion somewhere along the sequence. Hopefully, these would be included in the reference, though it could be that not all are. [TCTA]₇TCA[TCTA]₂ is an example of such an allele. Under existing alignment protocols, any read ending after the [TCTA]₇ and before the final [TCTA], will align to [flank1][TCTA]₇TCAGCTA, making an improper call.

### Alignment Methods

The methods provided herein allow for determining the length of a polymorphic repetitive DNA element having a repeat region situated between a first conserved flanking region and a second conserved flanking region. One method comprises providing a data set comprising at least one sequence read of a polymorphic repetitive DNA element; providing a reference sequence comprising the first conserved flanking region and the second conserved flanking region; aligning a portion of the first flanking region of the reference sequence to the sequence read; aligning a portion of the second flanking region of the reference sequence to the sequence read; and determining the length and/or sequence of the repeat region. In typical methods, one or more steps in the method are performed using a suitably programmed computer.

As used herein, the term "sequence read" refers to sequence data for which the length and/or identity of the repetitive element are to be determined. The sequence read can comprise all of the repetitive element, or a portion thereof. The sequence read can further comprise a conserved flanking region on one end of the repetitive element (e.g., a 5' flanking region). The sequence read can further comprise an additional conserved flanking region on another end of the repetitive element (e.g., a 3' flanking region). In typical embodiments, the sequence read comprises sequence data from a PCR amplicon having a forward and reverse primer sequence. The sequence data can be obtained from any suitable sequence methodology. The sequencing read can be, for example, from a sequencing-by-synthesis (SBS) reaction, a sequencing-by-ligation reaction, or any other suitable sequencing methodology for which it is desired to determine the length and/or identity of a repetitive element. The sequence read can be a consensus sequence derived from multiple sequence reads. In certain embodiments, providing a reference sequence comprises identifying a locus of interest based upon the primer sequence of the PCR amplicon.

As used herein, the term "polymorphic repetitive DNA element" refers to any repeating DNA sequence, and the methods provided herein can be used to align the corresponding flanking regions of any such repeating DNA sequence. The methods presented herein can be used for any repeat region. The methods presented herein can be used for any region which is difficult to align, regardless of the repeat class. The method presented herein are especially useful for a region having conserved flanking regions. Additionally or alternatively, the methods presented herein are especially useful for sequencing reads which span the entire repeat region including at least a portion of each flanking region. In typical embodiments, the repetitive DNA element is a variable number tandem repeat (VNTR). VNTRs are polymorphisms where a particular sequence is repeated at that locus numerous times. Some VNTRs include minisatellites, and microsatellites, also known as simple sequence repeats (SSRs) or short tandem repeats (STRs). In some embodiments, the repetitive sequence is typically less than 20 base pairs, although larger repeating units can be aligned. For example, in typical embodiments, the repeating unit can be 2, 3, 4, 5, 6, 7, 8, 9, 10, 11, 12, 13, 14, 15, 16, 17, 18, 19, 20 or more nucleotides, and can be repeated up to 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 55, 60, 65, 70, 75, 80, 85, 90, 95 or up to at least 100 times or more. In certain embodiments, the polymorphic repetitive DNA element is an STR. In some methods, the STR is used for forensic purposes. In typical methods for forensic applications, for example, the polymorphic repetitive DNA element comprises tetra- or penta-nucleotide repeat units, however, the methods provided herein are suitable for any length of repeating unit. In certain methods, the repeat region is a short tandem repeat (STR) such as, for example, a STR selected from the CODIS autosomal STR loci, CODIS Y-STR loci, EU autosomal STR loci, EU Y-STR loci and the like. As an example, the CODIS (Combined DNA Index System) database is a set of core STR loci for identified by the FBI laboratory and includes 13 loci: CSF1PO, FGA, TH01, TPOX, VWA, D3S1358, D5S818, D7S820, D8S1179, D13S317, D16S539, D18S51 and D21S11. Additional STRs of interest to the forensic community and which can be aligned using the methods and systems provided herein include PENTA D and PENTA E. The methods and systems presented herein can be applied to any repetitive DNA element and are not limited to the STRs described above. As used herein, the term "reference sequence" refers to a known sequence which acts as a scaffold against which a sample sequence can be aligned. In typical embodiments of the methods and systems provided herein, the reference sequence comprises at least a first conserved flanking region and a second conserved flanking region. The term "conserved flanking region" refers to a region of sequence outside the repeat region. The region is typically conserved across many alleles, even though the repeat region may be polymorphic. A conserved flanking region as used herein typically will be of higher complexity than the repeat region. In typical embodiments, a single reference sequence can be used to align all alleles within a locus. In some embodiments, more than one reference sequence is used to align all alleles within a locus because of variation within the flanking region. For example, the repeat region for Amelogenin has differences in the flanks between X and Y, although a single reference can represent the repeat region if a longer region is included in the reference.

In methods presented herein a portion of a flanking region of a reference sequence is aligned to the sequence read. Aligning is performed by determining a location of the conserved flanking region and then conducting a sequence alignment of that portion of the flanking region with the corresponding portion of the sequence read. Aligning of a portion of a flanking region is performed according to known alignment methods. In certain methods, the aligning a portion of the flanking region in one or both of steps (c) and (d) comprises: (i) determining a location of a conserved flanking region on the read by using exact k-mer matching of a seeding region which overlaps or is adjacent to the repeat region; and (ii) aligning the flanking region to the sequence read. In some methods, the aligning can further comprise aligning both the flanking sequence and a short adjacent region comprising a portion of the repeat region.

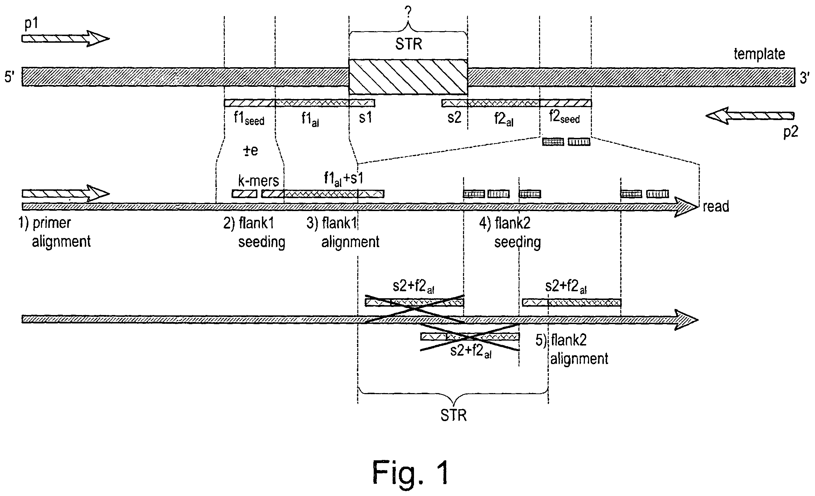

An example of this approach is illustrated in Figure 1. An amplicon ("template") is shown in Figure 1 having a STR of unknown length and/or identity. As shown in Figure 1, an initial primer alignment is conducted to identify the locus of interest, in this case an STR. The primers are illustrated as p1 and p2, which are the primer sequences that were used to generate the amplicon. In the embodiment shown in Figure 1, p1 alone is used during the primer alignment step. In some embodiments, p2 alone is used for primer alignment. In other embodiments, both p1 and p2 are used for primer alignment.

Following primer alignment, flank 1 is aligned, designated in Figure 1 as f1ₐₗ. Flank 1 alignment can be preceded by seeding of flank 1, designated in Figure 1 as f1_{seed}. Flank 1 seeding to correct for a small number (e) of indels between the beginning of the read and the STR. The seeding region may be directly next to the beginning of the STR, or may be offset (as in figure) to avoid low-complexity regions. Seeding can be done by exact k-mer matching.

Flank1 alignment proceeds to determine the beginning position of the STR sequence. If the STR pattern is conserved enough to predict the first few nucleotides (s1), these are added to the alignment for improved accuracy.

Since the length of the STR is unknown, an alignment is performed for flank2 as follows. Flank2 seeding is performed to quickly find out possible end positions of the STR. As the seeding for flank 1, the seeding may be offset to avoid low-complexity regions and mis-alignment. Any flank 2 seeds that fail to align are discarded. Once flank2 properly aligns, the end position (s2) of the STR can be determined, and the length of the STR can be calculated.

The seeding region can be directly adjacent to the repeat region and/or comprises a portion of the repeat region. In some methods, the location of the seeding region will depend on the complexity of the region directly adjacent to the repeat region. The beginning or end of an STR may be bounded by sequence that comprises additional repeats or which has low complexity. Thus, it can be advantageous to offset the seeding of the flanking region in order to avoid regions of low complexity. As used herein, the term "low-complexity" refers to a region with sequence that resembles that of the repeat sequence. Additionally or alternatively, a low-complexity region incorporates a low diversity of nucleotides. For example, in some embodiments, a low-complexity region comprises sequence having more than 30%, 40%, 50%, 60%, 70% or more than 80% sequence identity to the repeat sequence. In typical embodiments, the low-complexity region incorporates each of the four nucleotides at a frequency of less than 20%, 15%, 10% or less than 5% of all the nucleotides in the region. Any suitable method may be utilized to determine a region of low-complexity. Methods of determining a region of low-complexity are known in the art, as exemplified by the methods disclosed in Morgulis et al., (2006) Bioinformatics. 22(2):134-41. For example, as described in Morgulis et al., an algorithm such as DUST may be used to identify regions within a given nucleotide sequence that have low complexity.

In some embodiments, the seeding is offset from the start of the STR by at least 1, 2, 3, 4, 5, 6, 7, 8, 9, 10, 15, 20, 25, 30, 35, 40 or more nucleotides. In some embodiments, the flanking region is evaluated to identify a region of high complexity. As used herein, the term "high-complexity region" refers to a region with sequence that is different enough from that of repeat that it removes possibilities of mis-alignments. Additionally or alternatively, a high complexity region incorporates a variety of nucleotides. For example, in some embodiments, a high-complexity region comprises sequence having less than 80%, 70%, 60%, 50%, 40%, 30%, 20% or less than 10% identity to the repeat sequence. In typical embodiments, the high-complexity region incorporates each of the four nucleotides at a frequency of at least 10%, 15%, 20%, or at least 25% of all the nucleotides in the region.

As used herein, the term "exact k-mer matching" refers to a method to find optimal alignment by using a word method where the word length is defined as having a value k. In some embodiments, the value of k is 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more nucleotides in length. In typical embodiments, k has a value of between 5 and 30 nucleotides in length. In some typical embodiments, k has a value of between 5 and 16 nucleotides in length. In certain embodiments, k is chosen on-line. For example, if a flank region is short (primer close to the STR), k is reduced appropriately. In typical embodiments, k is chosen so as to guarantee finding all matches with edit distance e. Word methods identify a series of short, nonoverlapping subsequences ("words") in the query sequence that are then matched to candidate database sequences. The relative positions of the word in the two sequences being compared are subtracted to obtain an offset; this will indicate a region of alignment if multiple distinct words produce the same offset. Only if this region is detected do these methods apply more sensitive alignment criteria; thus, many unnecessary comparisons with sequences of no appreciable similarity are eliminated. Methods of performing k-mer matching, including exact k-mer matching, are well known in the art, as exemplified by the disclosure of Lipman, et al., (1985) Science 227:1435-41, and of Altschul, et al., (1990) Journal of Molecular Biology 215:403-410.

In certain embodiments, providing a reference sequence comprises identifying a locus of interest based upon the primer sequence of an amplicon. As used herein, the term "amplicon" refers to any suitable amplification product for which is a sequence is obtained. Typically, the amplification product is a product of a selective amplification methodology, using target-specific primers, such as PCR primers. In certain embodiments, the sequence data is from a PCR amplicon having a forward and reverse primer sequence. In some embodiments, selectively amplifying can include one or more non-selective amplification steps. For example, an amplification process using random or degenerate primers can be followed by one or more cycles of amplification using target-specific primers. Suitable methods for selective amplification include, but are not limited to, the polymerase chain reaction (PCR), strand displacement amplification (SDA), transcription mediated amplification (TMA) and nucleic acid sequence based amplification (NASBA), as described in U.S. Patent No. 8,003,354. The above amplification methods can be employed to selectively amplify one or more nucleic acids of interest. For example, PCR, including multiplex PCR, SDA, TMA, NASBA and the like can be utilized to selectively amplify one or more nucleic acids of interest. In such embodiments, primers directed specifically to the nucleic acid of interest are included in the amplification reaction. Other suitable methods for amplification of nucleic acids can include oligonucleotide extension and ligation, rolling circle amplification (RCA) (Lizardi et al., Nat. Genet. 19:225-232 (1998)) and oligonucleotide ligation assay (OLA) (See generally U.S. Pat. Nos. 7,582,420, 5,185,243, 5,679,524 and 5,573,907; EP 0 320 308 B1; EP 0 336 731 B1; EP 0 439 182 B1; WO 90/01069; WO 89/12696; and WO 89/09835) technologies. It will be appreciated that these amplification methodologies can be designed to selectively amplify a target nucleic acid of interest. For example, in some embodiments, the selective amplification method can include ligation probe amplification or oligonucleotide ligation assay (OLA) reactions that contain primers directed specifically to the nucleic acid of interest. In some embodiments, the selective amplification method can include a primer extension-ligation reaction that contains primers directed specifically to the nucleic acid of interest. As a non-limiting example of primer extension and ligation primers that can be specifically designed to amplify a nucleic acid of interest, the amplification can include primers used for the GoldenGate™ assay (Illumina, Inc., San Diego, CA), as described in U.S. Pat. No. 7,582,420 The present methods are not limited to any particular amplification technique and amplification techniques described herein are exemplary only with regard to methods and embodiments of the present disclosure.

Primers for amplification of a repetitive DNA element typically hybridize to the unique sequences of flanking regions. Primers can be designed and generated according to any suitable methodology. Design of primers for flanking regions of repeat regions is well known in the art, as exemplified by Zhi, et al. (2006) Genome Biol, 7(1):R7. For example, primers can be designed manually. This involves searching the genomic DNA sequence for microsatellite repeats, which can be done by eye or by using automated tools such as RepeatMasker software. Once the repeat regions and the corresponding flanking regions are determined, the flanking sequences can be used to design oligonucleotide primers which will amplify the specific repeat in a PCR reaction.

### Systems

Also presented herein is a system for determining the length of a polymorphic repetitive DNA element having a repeat region situated between a first conserved flanking region and a second conserved flanking region, the system comprising: a processor; and a program for determining the length of a polymorphic repetitive DNA element, the program comprising instructions for: (a) providing a data set comprising at least one sequence read of the polymorphic repetitive DNA element; (b) providing a reference sequence comprising the first conserved flanking region and the second conserved flanking region; (c) aligning a portion of the first flanking region of the reference sequence to the sequence read; (d) aligning a portion of the second flanking region of the reference sequence to the sequence read; and (e) determining the length and/or sequence of the repeat region; wherein at least steps (c), (d) and (e) are performed using a suitably programmed computer. In some systems, the aligning a portion of the flanking region in one or both of steps (c) and (d) comprises: (i) determining a location of a conserved flanking region on the read by using exact k-mer matching of a seeding region which overlaps or is adjacent to the repeat region; and (ii) aligning the flanking region to the sequence read. In some systems, the aligning can further comprise aligning both the flanking sequence and a short adjacent region comprising a portion of the repeat region.

A system capable of carrying out a method set forth herein can be, but need not be, integrated with a sequencing device. Rather, a stand-alone system or a system integrated with other devices is also possible. A system capable of carrying out a method set forth herein, whether integrated with detection capabilities or not, can include a system controller that is capable of executing a set of instructions to perform one or more steps of a method, technique or process set forth herein. Optionally, the instructions can further direct the performance of steps for detecting nucleic acids. A useful system controller may include any processor-based or microprocessor-based system, including systems using microcontrollers, reduced instruction set computers (RISC), application specific integrated circuits (ASICs), field programmable gate array (FPGAs), logic circuits, and any other circuit or processor capable of executing functions described herein. A set of instructions for a system controller may be in the form of a software program. As used herein, the terms "software" and "firmware" are interchangeable, and include any computer program stored in memory for execution by a computer, including RAM memory, ROM memory, EPROM memory, EEPROM memory, and non-volatile RAM (NVRAM) memory. The software may be in various forms such as system software or application software. Further, the software may be in the form of a collection of separate programs, or a program module within a larger program or a portion of a program module. The software also may include modular programming in the form of object-oriented programming.

### EXAMPLE 1

### Alignment of the locus D18S51

This example describes alignment of the locus D18S51 according to one embodiment. Some loci have flanking sequences which are low-complexity and resemble the STR repeat sequence. This can cause the flanking sequence to be misaligned (sometimes to the STR sequence itself) and thus the allele can be be miscalled. An example of a troublesome locus is D18S51. The repeat motif is **[AGAA] n AAAG AGAGAG.** The flanking sequence is shown below with the low-complexity "problem" sequence underlined:
GAGACCTTGTCTC **(STR) GAAAGAAAGAGAAAAAGAAAAGAAA**TAGTAGCAACTGTTAT

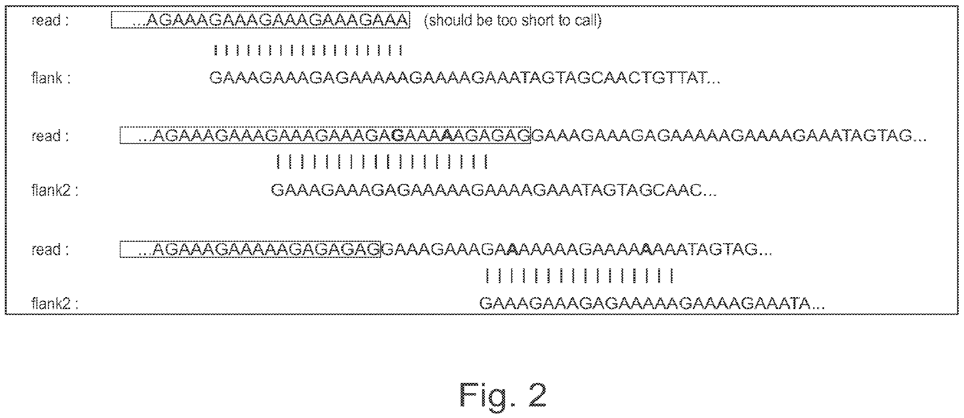

If the flanking region immediately adjacent to the STR were used to seed the alignment, k-mers would be generated such as GAAAG, AAAGAA, AGAGAAA, which map to the STR sequence. This deters performance since many possibilities are obtained from the seeding, but most importantly, the approach creates mis-alignments, such as those shown in Figure 2. In the sequences shown in Figure 2, the true STR sequence is highlighted, the STR sequence resulting from the mis-alignment is underlined and read errors are shown in bold.

For these low-complexity flanks, it was ensured that the seeding regions are not in the low-complexity region by pushing them further away from the STR sequence. While this requires longer reads to call the STR, it ensures high-accuracy and prevents mis-alignment of the flanking region to STR sequence (or other parts of the flank). The low-complexity flank is still aligned to the read to find the ending position of the STR but because the alignment is seeded with high-complexity sequence it has to be in the correct position.

### EXAMPLE 2

### Alignment of the locus Penta-D by short STR Sequence Addition

A set of Penta-D sequences tended to have STRs that were 1 nt shorter than expected. Upon further inspection, it was discovered that both flanks contained poly-A stretches and sequencing / amplification errors often removed one of the A's in those stretches. As shown in the sequence below, homopolymeric A stretches are found on both flanks.

| |
|---|
| ...CAAGAAAG**AAAAAAAA**G **[AAAGA]n AAAAA**CGAAGGGG**AAAAAAA**GAGAAT**...** |

A read error causing a deletion in the first flank would yield to two equally viable alignments:

| | |
|---|---|
| **read: ...**CAAGAAAG**AAAAAAA-**GA**...** | |
| flank: **...**CAAGAAAG**AAAAAAAA**G- | (2 indels) |
| **read: ...**CAAGAAAG**AAAAAAA**GA**...** | (2 mismatches) |
| flank: ...CAAGAAAG**AAAAAAAA**G | |

Enforcing the base closest to the STR to be a match did not work because one of the flanks in one of the STRs ended up having a SNP in it, causing us to reconsider that method all together. It was discovered that adding just 2 bases of the STR sequence solved the issue:

| | |
|---|---|
| **read: ...**CAAGAAAG**AAAAAAA-**GAA | |
| **flank: ...**CAAGAAAG**AAAAAAAA**GAA | (1 indel) ✔ |
| **read: ...**CAAGAAAG**AAAAAAA**G-AA | (1 indel + 1 mismatch) |
| **flank: ...**CAAGAAAG**AAAAAAAA**GAA | |

### EXAMPLE 3

### Analysis of Mixture of DNA Samples

A mixture of samples was analyzed using the methods provided herein to make accurate calls for each locus in a panel of forensic STRs. For each locus, the number reads corresponding to each allele and to each different sequence for that allele were counted.

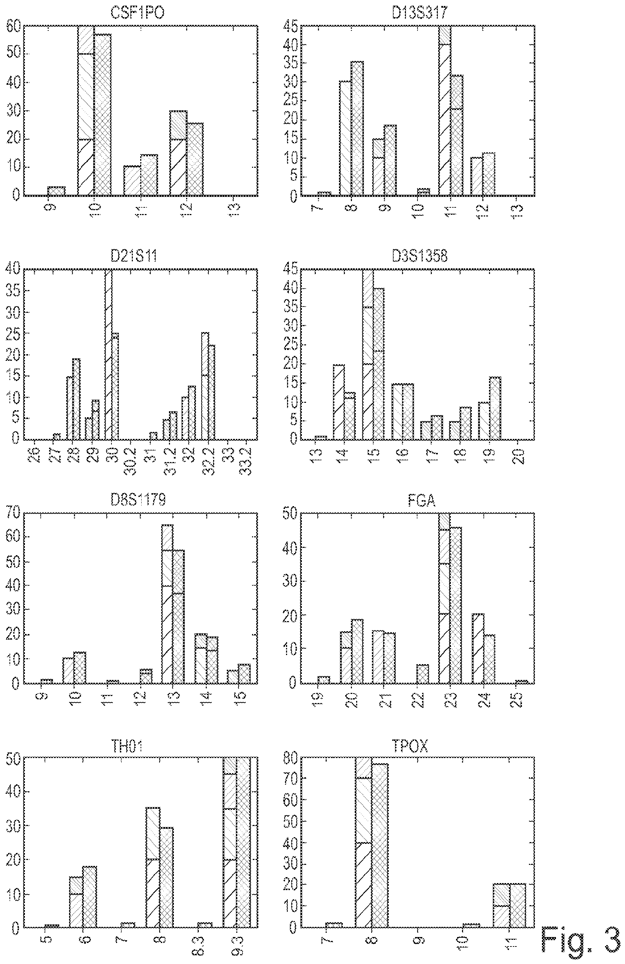

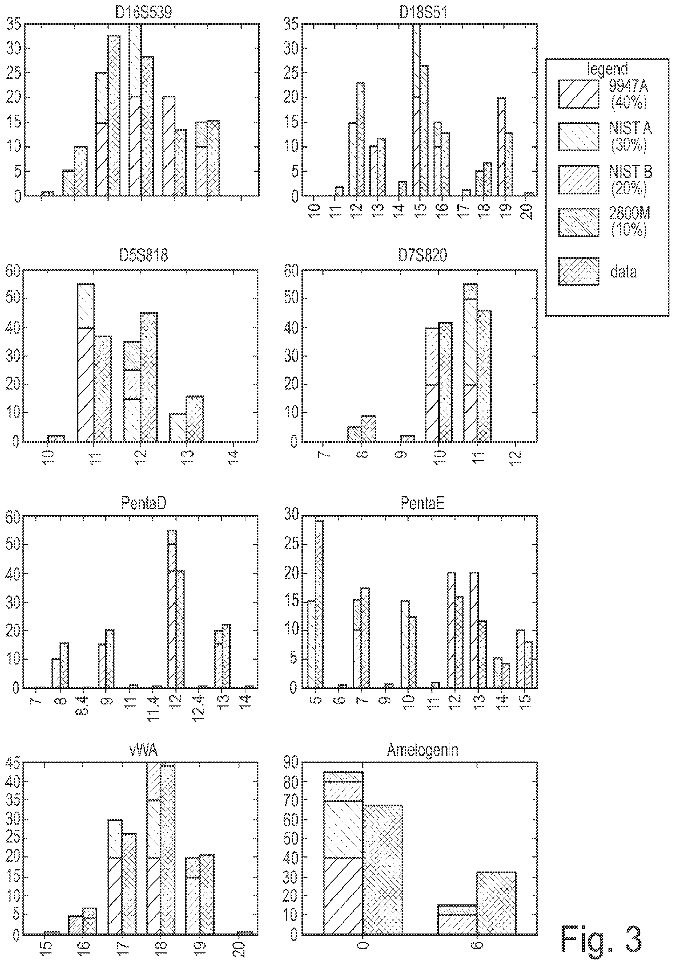

Typical results are shown in Figure 3. As shown, the bar on the right of each pair represents the actual data obtained, indicating the proportion of reads for each allele. Different shades represent different sequences. Alleles with less than 0.1% of the locus read count and sequences with less than 1 % of the allele count are omitted. The bar on the left side of each pair represents the theoretical proportions (no stutter). Different shades represent different control DNA in the input as indicated in the legend. In Figure 3, the x-axis is in order allele, and the Y axis indicates proportion of reads with the indicated allele.

As shown in the Figure, the STR calling approach using the methods presented herein achieved surprisingly accurate calls for each allele in the panel.

### EXAMPLE 4

### Analysis of Forensic STR Panel

A panel of 15 different loci were analyzed in 5 different samples. The samples were obtained from Promega Corp, and included samples 9947A, K562, 2800M, NIST: A and B (SRM 2391c). The loci were chosen from the CODIS STR forensic markers and included CSF1PO, D3S1358, D7S820, D16S539, D18S51, FGA, PentaE, TH01, vWA, D5S818, D8S1179, D13S317, D21S11, PentaD and TPOX using the alignment method presented herein. Briefly, the markers were amplified using standard primers, as set forth in Krenke, et al. (2002) J. Forensic Sci. 47(4): 773-785. The amplicons were pooled and sequencing data was obtained using 1x460 cycles on a MiSeq sequencing instrument (Illumina, San Diego, CA).

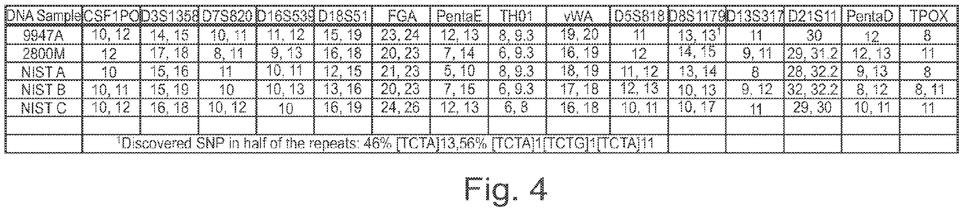

Alignment was performed according to the methods presented herein. As set forth in Fig. 4, 100% concordance for these control samples was shown compared to control data. In addition, this method identified a previously-unknown SNP in one of the samples for marker D8S1179, further demonstrating the powerful tool of sequence-based STR analysis when combined with the alignment methods provided herein.

The term comprising is intended herein to be open-ended, including not only the recited elements, but further encompassing any additional elements.

A number of embodiments have been described. Nevertheless, it will be understood that various modifications may be made.

## Claims

1. A method for determining the length and/or sequence of a polymorphic repetitive DNA element having a repeat region situated between a first conserved flanking region and a second conserved flanking region, the method comprising:
(a) providing a data set comprising at least one sequence read of the polymorphic repetitive DNA element;
(b) providing a reference sequence comprising the first conserved flanking region and the second conserved flanking region;
(c) aligning a portion of the first flanking region of the reference sequence to the sequence read;
(d) aligning a portion of the second flanking region of the reference sequence to the sequence read; and
(e) determining the length and/or sequence of the repeat region; wherein at least steps (c), (d) and (e) are performed using a suitably programmed computer;
wherein the aligning a portion of the flanking region in one or both of steps (c) and (d) comprises:
(i) determining a location of a conserved flanking region on the read by using exact k-mer matching of a seeding region which overlaps or is adjacent to the repeat region; and
(ii) aligning the flanking region to the sequence read;
wherein the seeding region comprises a high-complexity region of the conserved flanking region, the high-complexity region comprising sequence that is sufficiently distinct from the repeat region so as to avoid mis-alignment.

2. The method of claim 1, further comprising aligning both the flanking sequence and a short adjacent region comprising a portion of the repeat region.

3. The method of claim 1, wherein the high-complexity region comprises a sequence having a diverse mixture of bases.

4. The method of claim 1, wherein the seeding region avoids low-complexity regions of the conserved flanking region.

5. The method of claim 4, the low-complexity region comprising sequence that substantially resembles that of the repeat sequence.

6. The method of claim 4, the low-complexity region comprising sequence having a mixture of bases with low diversity.

7. The method of claim 1, wherein the seeding region is directly adjacent to the repeat region.

8. The method of claim 1, wherein the seeding region comprises a portion of the repeat region.

9. The method of claim 1, wherein the seeding region is offset from the repeat region.

10. The method of claim 1, wherein the at least one sequence read in the data set comprises a consensus sequence derived from multiple sequence reads.

11. The method of claim 1, wherein providing a reference sequence comprises identifying a locus of interest based upon a primer sequence of a PCR amplicon.

12. A system for determining the length and/or sequence of a polymorphic repetitive DNA element having a repeat region situated between a first conserved flanking region and a second conserved flanking region, the system comprising:
a processor; and
a program for determining the length and/or sequence of a polymorphic repetitive DNA element, the program comprising instructions for the processor to perform the following steps:
(a) providing a data set comprising at least one sequence read of the polymorphic repetitive DNA element;
(b) providing a reference sequence comprising the first conserved flanking region and the second conserved flanking region;
(c) aligning a portion of the first flanking region of the reference sequence to the sequence read;
(d) aligning a portion of the second flanking region of the reference sequence to the sequence read; and
(e) determining the length and/or sequence of the repeat region;
wherein the aligning a portion of the flanking region in one or both of steps (c) and (d) comprises:
(i) determining a location of a conserved flanking region on the read by using exact k-mer matching of a seeding region which overlaps or is adjacent to the repeat region; and
(ii) aligning the flanking region to the sequence read;
wherein the seeding region comprises a high-complexity region of the conserved flanking region, the high-complexity region comprising sequence that is sufficiently distinct from the repeat region so as to avoid mis-alignment.

## Patentansprüche

1. Ein Verfahren zum Bestimmen der Länge und/oder der Sequenz eines polymorphen repetitiven DNA-Elements, das einen Wiederholungsbereich aufweist, der sich zwischen einem ersten konservierten flankierenden Bereich und einem zweiten konservierten flankierenden Bereich befindet, wobei das Verfahren Folgendes beinhaltet:
(a) Bereitstellen eines Datensatzes, der mindestens einen Sequenzabschnitt des polymorphen repetitiven DNA-Elements beinhaltet;
(b) Bereitstellen einer Referenzsequenz, die den ersten konservierten flankierenden Bereich und den zweiten konservierten flankierenden Bereich beinhaltet;
(c) Ausrichten eines Teils des ersten flankierenden Bereichs der Referenzsequenz nach dem Sequenzabschnitt;
(d) Ausrichten eines Teils des zweiten flankierenden Bereichs der Referenzsequenz nach dem Sequenzabschnitt; und
(e) Bestimmen der Länge und/oder der Sequenz des Wiederholungsbereichs; wobei mindestens die Schritte (c), (d) und (e) unter Verwendung eines geeignet programmierten Computers durchgeführt werden;
wobei das Ausrichten eines Teils des flankierenden Bereichs in einem oder beiden der Schritte (c) und (d) Folgendes beinhaltet:
(i) Bestimmen einer Stelle eines konservierten flankierenden Bereichs auf dem Abschnitt durch das Verwenden genauer k-Mer-Anpassung eines Impfbereichs, der den Wiederholungsbereich überlappt oder an diesen angrenzt; und
(ii) Ausrichten des flankierenden Bereichs nach dem Sequenzabschnitt;
wobei der Impfbereich einen Bereich hoher Komplexität des konservierten flankierenden Bereichs beinhaltet, wobei der Bereich hoher Komplexität eine Sequenz beinhaltet, die sich ausreichend klar von dem Wiederholungsbereich unterscheidet, um eine fehlerhafte Ausrichtung zu vermeiden.

2. Verfahren gemäß Anspruch 1, das ferner das Ausrichten sowohl der flankierenden Sequenz als auch eines kurzen angrenzenden Bereichs, beinhaltend einen Teil des Wiederholungsbereichs, beinhaltet.

3. Verfahren gemäß Anspruch 1, wobei der Bereich hoher Komplexität eine Sequenz beinhaltet, die eine vielfältige Mischung von Basen aufweist.

4. Verfahren gemäß Anspruch 1, wobei der Impfbereich Bereiche niedriger Komplexität des konservierten flankierenden Bereichs vermeidet.

5. Verfahren gemäß Anspruch 4, wobei der Bereich niedriger Komplexität eine Sequenz beinhaltet, die im Wesentlichen der der Wiederholungssequenz ähnelt.

6. Verfahren gemäß Anspruch 4, wobei der Bereich niedriger Komplexität eine Sequenz beinhaltet, die eine Mischung von Basen mit niedriger Vielfalt aufweist.

7. Verfahren gemäß Anspruch 1, wobei der Impfbereich direkt an den Wiederholungsbereich angrenzt.

8. Verfahren gemäß Anspruch 1, wobei der Impfbereich einen Teil des Wiederholungsbereichs beinhaltet.

9. Verfahren gemäß Anspruch 1, wobei der Impfbereich zu dem Wiederholungsbereich versetzt ist.

10. Verfahren gemäß Anspruch 1, wobei der mindestens eine Sequenzabschnitt in dem Datensatz eine Konsensussequenz beinhaltet, die von mehreren Sequenzabschnitten abgeleitet ist.

11. Verfahren gemäß Anspruch 1, wobei das Bereitstellen einer Referenzsequenz das Identifizieren eines Orts von Interesse, basierend auf einer Primärsequenz eines PCR-Amplikons, beinhaltet.

12. Ein System zum Bestimmen der Länge und/oder der Sequenz eines polymorphen repetitiven DNA-Elements, das einen Wiederholungsbereich aufweist, der sich zwischen einem ersten konservierten flankierenden Bereich und einem zweiten konservierten flankierenden Bereich befindet, wobei das System Folgendes beinhaltet:
einen Prozessor; und
ein Programm zum Bestimmen der Länge und/oder der Sequenz eines polymorphen repetitiven DNA-Elements, wobei das Programm Anweisungen für den Prozessor beinhaltet, die folgenden Schritte durchzuführen:
(a) Bereitstellen eines Datensatzes, der mindestens einen Sequenzabschnitt des polymorphen repetitiven DNA-Elements beinhaltet;
(b) Bereitstellen einer Referenzsequenz, die den ersten konservierten flankierenden Bereich und den zweiten konservierten flankierenden Bereich beinhaltet;
(c) Ausrichten eines Teils des ersten flankierenden Bereichs der Referenzsequenz nach dem Sequenzabschnitt;
(d) Ausrichten eines Teils des zweiten flankierenden Bereichs der Referenzsequenz nach dem Sequenzabschnitt; und
(e) Bestimmen der Länge und/oder der Sequenz des Wiederholungsbereichs; wobei das Ausrichten eines Teils des flankierenden Bereichs in einem oder beiden der Schritte (c) und (d) Folgendes beinhaltet:
(i) Bestimmen einer Stelle eines konservierten flankierenden Bereichs auf dem Abschnitt durch das Verwenden genauer k-Mer-Anpassung eines Impfbereichs, der den Wiederholungsbereich überlappt oder an diesen angrenzt; und
(ii) Ausrichten des flankierenden Bereichs nach dem Sequenzabschnitt;
wobei der Impfbereich einen Bereich hoher Komplexität des konservierten flankierenden Bereichs beinhaltet, wobei der Bereich hoher Komplexität eine Sequenz beinhaltet, die sich ausreichend klar von dem Wiederholungsbereich unterscheidet, um eine fehlerhafte Ausrichtung zu vermeiden.

## Revendications

1. Une méthode pour déterminer la longueur et/ou séquence d'un élément ADN répétitif polymorphe ayant une région de répétition située entre une première région flanquante conservée et une deuxième région flanquante conservée, la méthode comprenant :
(a) le fait de fournir une ensemble de données comprenant au moins une lecture de séquence de l'élément ADN répétitif polymorphe ;
(b) le fait de fournir une séquence de référence comprenant la première région flanquante conservée et la deuxième région flanquante conservée ;
(c) l'alignement d'une partie de la première région flanquante de la séquence de référence par rapport à la lecture de séquence ;
(d) l'alignement d'une partie de la deuxième région flanquante de la séquence de référence par rapport à la lecture de séquence ; et
(e) la détermination de la longueur et/ou séquence de la région de répétition ;
dans laquelle au moins les étapes (c), (d) et (e) sont effectuées en utilisant un ordinateur programmé de façon appropriée ;
dans laquelle l'alignement d'une partie de la région flanquante dans l'une des étapes (c) et (d), ou les deux, comprend :
(i) la détermination d'un emplacement d'une région flanquante conservée sur la lecture en utilisant une correspondance de k-mers exacte d'une région de seeding qui chevauche ou qui est adjacente à la région de répétition ; et
(ii) l'alignement de la région flanquante par rapport à la lecture de séquence ;
dans laquelle la région de seeding comprend une région de complexité élevée de la région flanquante conservée, la région de complexité élevée comprenant une séquence qui est suffisamment distincte de la région de répétition de façon à éviter un désalignement.

2. La méthode de la revendication 1, comprenant en outre l'alignement à la fois de la séquence flanquante et d'une région adjacente courte comprenant une partie de la région de répétition.

3. La méthode de la revendication 1, dans laquelle la région de complexité élevée comprend une séquence ayant un mélange divers de bases.

4. La méthode de la revendication 1, dans laquelle la région de seeding évite des régions de complexité faible de la région flanquante conservée.

5. La méthode de la revendication 4, la région de complexité faible comprenant une séquence qui ressemble substantiellement à celle de la séquence de répétition.

6. La méthode de la revendication 4, la région de complexité faible comprenant une séquence ayant un mélange de bases avec une faible diversité.

7. La méthode de la revendication 1, dans laquelle la région de seeding est directement adjacente à la région de répétition.

8. La méthode de la revendication 1, dans laquelle la région de seeding comprend une partie de la région de répétition.

9. La méthode de la revendication 1, dans laquelle la région de seeding est décalée relativement à la région de répétition.

10. La méthode de la revendication 1, dans laquelle l'au moins une lecture de séquence dans l'ensemble de données comprend une séquence consensus dérivée de lectures de séquences multiples.

11. La méthode de la revendication 1, dans laquelle le fait de fournir une séquence de référence comprend l'identification d'un locus d'intérêt sur la base d'une séquence d'amorce d'un amplicon de PCR.

12. Un système pour déterminer la longueur et/ou séquence d'un élément ADN répétitif polymorphe ayant une région de répétition située entre une première région flanquante conservée et une deuxième région flanquante conservée, le système comprenant :
un processeur ; et
un programme pour déterminer la longueur et/ou séquence d'un élément ADN répétitif polymorphe, le programme comprenant des instructions pour que le processeur effectue les étapes suivantes :
(a) le fait de fournir une ensemble de données comprenant au moins une lecture de séquence de l'élément ADN répétitif polymorphe ;
(b) le fait de fournir une séquence de référence comprenant la première région flanquante conservée et la deuxième région flanquante conservée ;
(c) l'alignement d'une partie de la première région flanquante de la séquence de référence par rapport à la lecture de séquence ;
(d) l'alignement d'une partie de la deuxième région flanquante de la séquence de référence par rapport à la lecture de séquence ; et
(e) la détermination de la longueur et/ou séquence de la région de répétition ;
dans laquelle l'alignement d'une partie de la région flanquante dans l'une des étapes (c) et (d), ou les deux, comprend :
(i) la détermination d'un emplacement d'une région flanquante conservée sur la lecture en utilisant une correspondance de k-mers exacte d'une région de seeding qui chevauche ou qui est adjacente à la région de répétition ; et
(ii) l'alignement de la région flanquante par rapport à la lecture de séquence ;
dans laquelle la région de seeding comprend une région de complexité élevée de la région flanquante conservée, la région de complexité élevée comprenant une séquence qui est suffisamment distincte de la région de répétition de façon à éviter un désalignement.
